# FASCICULE DE BREVET EUROPEEN

(11) **EP 2 480 211 B1**
(45) Date de publication et mention de la délivrance du brevet: **29.11.2017**
(21) Numéro de dépôt: 10769012.5
(22) Date de dépôt: 22.09.2010
(51) Int. Cl.: A61K 9/14, A61K 9/16, A61K 31/722, A61K 33/26, A61K 33/30, A61K 33/34

(54) **FORME GALENIQUE CAPABLE D'ADSORBER DE MANIERE SPECIFIQUE LES MOLECULES INDESIRABLES PRESENTES DANS LE TUBE DISGESTIF**
GALENISCHE FORM ZUM GEZIELTEN ABSORBIEREN UNERWÜNSCHTER MOLEKÜLE IM VERDAUUNGSTRAKT
GALENIC FORM SUITABLE FOR ABSORBING, IN A SPECIFIC MANNER, THE UNDESIRABLE MOLECULES IN THE DIGESTIVE TRACT

(30) Priorité: 23.09.2009 FR 0956542
(43) Date de publication de la demande: 01.08.2012
(73) Titulaire: Centre National de la Recherche Scientifique (C.N.R.S.), 75794 Paris Cedex 16 (FR)
(72) Inventeur: FATTAL, Elias, 75012 Paris (FR); TSAPIS, Nicolas, 75014 Paris (FR); REYNAUD, Franceline, 88.306-180 Itajai (BR)
(74) Mandataire: Novagraaf Technologies
(86) Numéro de dépôt international: PCT/FR2010/051976
(87) Numéro de publication internationale: WO 2011/036400

(56) Documents cités:
- WO-A1-2006/122835
- WO-A2-2004/016248
- WO-A2-2007/132022
- FR-A1- 2 843 301
- US-A- 4 810 695
- US-A1- 2009 162 339
- BÜRGER, C. ET AL: "Cross-linking chitosan-Fe(III), an oral phosphate binder: studies in vitro and in vivo", INTERNATIONAL JOURNAL OF PHARMACEUTICS, vol. 223, no. 1-2, 31 juillet 2001 (2001-07-31), pages 29-33, XP002617691,
- KARA, M. ET AL: "Clinical and chemical interactions between iron preparations and ciprofloxacin.", BR J CLIN PHARMACOL., vol. 31, no. 3, mars 1991 (1991-03), pages 257-261, XP002617692,
- CHOW, L. ET AL: "pH Dependence of Chitosan Solubility Determined Using a Titration Method", The IADR/AADR/CADR 83rd General Session , 10 mars 2005 (2005-03-10), XP002617693, Extrait de l'Internet: URL:http://iadr.confex.com/iadr/2005Balt/t echprogram/abstract_60955.htm [extrait le 2011-01-20]

## Description

### Domaine technique

La présente invention se rapporte à une forme galénique comprenant des particules capables d'adsorber, de manière spécifique, les molécules indésirables présentes dans le tube digestif, son procédé de préparation et son utilisation notamment pour la fabrication d'un médicament destiné à la prévention ou au traitement des effets indésirables liés à un déséquilibre de la flore intestinale et/ou colique pouvant résulter par exemple d'une antibiothérapie.

Dans la description ci-dessous, les références entre crochets ([ ]) renvoient à la liste des références présentée à la fin du texte.

### Etat de la technique

L'administration orale des médicaments présente un intérêt par rapport à d'autres voies d'administration comme par exemple la voie parentérale. L'administration par voie parentérale est en général douloureuse et peut entraîner des complications. De plus, cette voie d'administration nécessite du matériel adapté, du personnel qualifié et des conditions d'asepsie empêchant tout apport exogène de micro-organismes (bactéries, parasites etc.).

Selon la nature du principe actif, une administration par voie orale peut être avantageuse. Cela permet aux principes actifs des médicaments de traverser l'estomac puis d'être absorbés au niveau de l'intestin grêle pour diffuser dans l'ensemble de l'organisme et traiter le foyer infectieux pour lequel ils ont été administrés. C'est le cas par exemple des antibiotiques.

Toutefois, une fraction des antibiotiques ingérés (dont l'importance varie avec les caractéristiques propres de chaque type d'antibiotique) n'est pas absorbée et continue sa progression jusqu'au colon avant d'être éliminée dans les selles. Ces antibiotiques résiduels sont rejoints, au niveau de l'intestin grêle, par une fraction des antibiotiques absorbés mais qui sont ré-excrétés dans le tube digestif par l'intermédiaire de l'élimination biliaire.

Cette fraction est d'importance variable en fonction du métabolisme et des voies d'élimination de chaque antibiotique. Enfin, pour certains antibiotiques, une fraction de la dose absorbée est éliminée directement par la muqueuse intestinale dans la lumière du tube digestif. Ainsi, que les antibiotiques aient été administrés par voie orale ou par voie parentérale, une fraction résiduelle active se retrouve généralement au niveau du tube digestif, en particulier du colon. Cela est vrai, à des degrés divers, pour la grande majorité des familles d'antibiotiques utilisés en thérapeutique, la seule exception notable étant la famille des aminoglycosides pour lesquels l'excrétion intestinale est négligeable. Pour les autres antibiotiques, l'excrétion intestinale d'une activité antibiotique résiduelle va avoir différentes conséquences toutes délétères. En effet, il existe au niveau du colon un écosystème bactérien complexe (plusieurs centaines d'espèces bactériennes différentes) et très dense (plus de 10 millions de bactéries par gramme de contenu colique) qui va être affecté par l'arrivée des résidus actifs d'antibiotiques.

La première conséquence de l'arrivée des résidus actifs d'antibiotiques est que les nombreuses bactéries qui peuplent le colon vont subir l'action de l'antibiotique. La grande majorité de ces bactéries est sensible à l'antibiotique et l'action de ce dernier aboutit à :
- un déséquilibre de la flore qui serait la cause principale des diarrhées banales suivant parfois la prise d'antibiotiques [1]. Ces diarrhées ne sont, en règle générale, pas graves et cessent rapidement soit spontanément soit à l'arrêt du traitement. Elles sont, toutefois, mal ressenties par les patients et ajoutent à l'inconfort de la maladie de base pour laquelle l'antibiotique a été prescrit. Des préparations pharmaceutiques à base de flores de remplacement, voire l'ingestion de yaourts, ont été préconisées pour combattre les déséquilibres de la flore colique suivant l'antibiothérapie. Aucune de ces attitudes n'a, cependant, fait la preuve de son efficacité de façon réellement convaincante ;
- une perturbation des fonctions de résistance à la colonisation par des bactéries exogènes (ou « effet barrière ») avec possibilités de risque accru d'infection, par exemple, intoxication alimentaire à salmonelles [2].

La deuxième conséquence de l'arrivée d'une activité antibiotique résiduelle sur la flore colique est la sélection de microorganismes résistants à l'antibiotique. Ces microorganismes peuvent être de divers type :
- il peut d'abord s'agir de bactéries pathogènes comme par exemple, *Clostridium difficile,* une espèce capable de sécréter des toxines causant de redoutables colites dites pseudomembraneuses [3] ;
- il peut aussi s'agir de microorganismes relativement peu pathogènes mais dont la multiplication peut amener à une infection de voisinage (Candidose vaginale ou cystite à *Escherichia Coli* résistant) ;
- il peut enfin s'agir de bactéries résistantes commensales non pathogènes mais dont la multiplication et l'élimination fécale va accroître la dissémination dans l'environnement. Or, ces bactéries commensales résistantes peuvent constituer une source importante de mécanismes de résistance pour des espèces pathogènes. Ce risque est actuellement considéré comme majeur en raison du caractère inquiétant de l'évolution vers la multirésistance de nombreuses espèces pathogènes pour l'homme.

Pour au moins toutes ces raisons, il subsiste un besoin de disposer d'un moyen pour adsorber et/ou inactiver les molécules résiduelles indésirables qui arrivent dans le tube digestif.

L'ingestion (volontaire et/ou accidentelle) de substances médicamenteuses ou domestiques, peut constituer un autre cas où l'adsorption et/ou l'inactivation des molécules indésirables est recherchée. En France, on dénombre chaque année plus de 130000 cas d'empoisonnement accidentels et volontaires, par des substances médicamenteuses ou domestiques. 10% de ces cas sont mortels. Habituellement, les patients sont amenés dans les services d'urgence et soumis à différents traitements. Bien que quelques substances toxiques aient des antidotes spécifiques, ceux-ci sont rares, leur efficacité n'est pas souvent prouvée et leur conservation est difficile. Il est à noter que seulement 10% des intoxications peuvent être traitées dans les hôpitaux. Dans la majorité des cas les cliniciens ne disposent que de solutions visant à limiter l'intoxication et assurer la survie du patient : vomissement provoqué, lavage gastrique, utilisation de charbon actif, de Terre de Foulon et traitement symptomatique. Ces traitements sont souvent lourds, coûteux et non dénués de risques. Dans de tels cas, disposer d'un moyen permettant le traitement en urgence des empoisonnements par voie orale devient une nécessité. Dans le cas des intoxications orales, les traitements mis en oeuvre à l'heure actuelle pour éviter le passage des toxiques dans le sang recourent soit au lavage gastrique (qui est proscrit dans le cas de produits corrosifs et qui présentent des risques au plan respiratoire), soit au charbon actif (dont l'efficacité n'a pas été totalement démontrée et qui, pour cette raison, est utilisée de manière variable selon les hôpitaux).

Par ailleurs, les résidus de médicaments, lorsqu'ils ne sont pas totalement dégradés dans l'organisme, sont excrétés dans les selles et les urines sous leur forme initiale ou sous la forme d'un ou de plusieurs métabolites. On considère aujourd'hui que l'impact environnemental des médicaments rejetés ainsi dans l'écosystème sera élevé.

Des formes galéniques basées sur la libération d'enzymes capables de dégrader des antibiotiques au niveau du colon, ont été proposées pour réduire la présence de molécules indésirables dans le tube digestif et/ou leur rejet dans l'écosystème. Toutefois, ces formes galéniques ne présentent pas toujours la stabilité recherchée tout le long du tube digestif ce qui peut entraîner la libération prématurée du principe actif.

D'autres formes galéniques permettant une délivrance d'adsorbants du type charbon, bentonite etc. au niveau du colon ont également été développés. L'inconvénient majeur de ce type de système réside dans le manque de spécificité de l'adsorption par rapport aux molécules adsorbées.

Qu'il s'agisse de substances toxiques, de résidus médicamenteux ou leurs métabolites, d'antibiotiques résiduels capables de modifier la flore colique et d'entraîner l'émergence de résistances, il existe un réel besoin à développer une forme galénique pour éliminer les molécules indésirables au niveau du tube digestif par adsorption, palliant les défauts, inconvénients et obstacles de l'art antérieur.

En particulier, il existe un réel besoin à développer une forme galénique à la fois simple à préparer, facilement reproductible et transposable à une plus grande échelle, permettant d'adsorber, de manière spécifique, les molécules indésirables présentes dans le tube digestif, notamment dans l'intestin grêle et/ou le colon.

Le document Journal of colloid and interface science 330 (2009), pages 29-37, décrit des nanoparticules magnétiques d'oxyde de fer recouvertes de chitosan et modifiées par de l'acide cétoglutarique, destinées à piéger les Cu2+ des eaux usées. Le document WO 2006/122835 A1 décrit des systèmes de délivrance comprenant des particules capables d'adsorber des résidus médicamenteux indésirables dans le tube digestive, les dites particules comprenant des adsorbants encapsulés dans des billes de pectine.

### Description de l'invention

La présente invention a précisément pour but de répondre à ce besoin en fournissant des formes galéniques comprenant des particules capables d'adsorber, de manière spécifique, les molécules indésirables présentes dans le tube digestif, lesdites particules comprenant un polymère cationique associé à au moins un ion métallique.

Il s'agit d'une forme galénique comprenant des particules capables d'adsorber, de manière spécifique, des molécules indésirables présentes dans le tube digestif, lesdites particules comprenant (i) un polymère cationique qui est le chitosan associé à (ii) au moins un ion métallique choisi dans le groupe comprenant, le cuivre, le zinc ou leur mélange ledit polymère cationique et ledit au moins un ion métallique formant un complexe.

Les composants du tube digestif peuvent être classés essentiellement en trois parties : l'oesophage, l'estomac et les intestins. Les intestins comprennent à leur tour l'intestin grêle, le gros intestin ou côlon et le rectum. Les formes galéniques selon l'invention peuvent être utilisées pour l'adsorption de molécules indésirables présentes au niveau de tous les composants du tube digestif.

Dans un mode particulier de réalisation, l'invention concerne les formes galéniques comprenant des particules capables d'adsorber, de manière spécifique, les molécules indésirables présentes dans les intestins, plus particulièrement dans l'intestin grêle et/ou le colon.

Au sens de l'invention, par « molécules indésirables », on entend les molécules indésirables pour l'organisme et/ou pour l'écosystème localisées dans le tube digestif, par exemple dans les intestins notamment dans l'intestin grêle et/ou le colon. A ce titre on peut citer comme molécules indésirables, de manière non limitative :
- les substances toxiques domestiques comme par exemple, les détergents, les substances rentrant dans la composition des pesticides ménagers, l'eau de javel, les détachants, les antirouilles (type Rubigine), les détartrants ;
- les résidus médicamenteux ou les métabolites des médicaments qui sont ingérés de manière volontaire et/ou accidentelle ;
- les résidus médicamenteux ou les métabolites des médicaments les plus employés en médecine humaine ou vétérinaire et que l'on retrouve dans les eaux, comme par exemple ceux indiqués à dans le rapport de l'Académie de Pharmacie [5] ;
- les antibiotiques résiduels, comme par exemple les beta-lactames, les quinolones notamment les fluoroquinolones, les aminoglucosides, les macrolides, les sulfamides, les antituberculeux, les tétracyclines.

Les formes galéniques selon l'invention comprennent des particules qui sont capables d'adsorber, de manière spécifique, les molécules indésirables se trouvant dans le tube digestif, par exemple dans les intestins notamment dans l'intestin grêle et/ou le colon. Dans le cadre de l'invention, le terme « spécifique » signifie que l'adsorption se fait de manière spécifique par rapport à un type de molécule donnée. En d'autres termes, les formes galéniques selon l'invention sont capables de distinguer les molécules indésirables à adsorber des autres molécules présentes dans le tube digestif, avantageusement dans les intestins et en particulier dans l'intestin grêle et/ou le colon.

Comme indiqué, les formes galéniques selon l'invention comprennent des particules capables d'adsorber, de manière spécifique, les molécules indésirables se trouvant dans le tube digestif. Ces particules comprennent un polymère cationique associé à au moins un ion métallique.

Le polymère cationique peut être, par exemple, choisi dans le groupe comportant les polyéthylèneimines, les polylysines, les polyarginines et les polysaccharides cationiques.

Dans un mode de réalisation particulier, le polysaccharide cationique est le chitosan.

Le chitosan est un polyoside composé de la distribution aléatoire de D-glucosamine liée en β-(1-4) (unité désacétylée) et de N-acétyl-D-glucosamine (unité acétylée). Le chitosan est produit par désacétylation chimique (en milieu alcalin) ou enzymatique de la chitine qui est l'un des principaux composants de l'exosquelette des insectes et d'autres arthropodes (crustacés) ou de l'endosquelette des céphalopodes (calamars, etc.) ou encore de la paroi des champignons. Contrairement à la chitine, le chitosan est insoluble en milieu alcalin et neutre mais soluble en milieu acide.

La frontière entre chitosan et chitine correspond à un degré d'acétylation (DA) de 50% : en-deçà le composé est nommé chitosan, au-delà, chitine. Le degré d'acétylation (DA) est le pourcentage d'unités acétylées par rapport au nombre d'unités totales, il peut être déterminé par spectrométrie infrarouge à transformée de Fourier (IR-TF) ou par un titrage par une base forte. Il est important de faire la distinction entre le degré d'acétylation (DA) et le degré de désacétylation (DD). L'un étant l'inverse de l'autre c'est-à-dire que par exemple du chitosan ayant un DD de 70%, possède 30% de groupements acétyles et 70% de groupements amines sur ses chaînes.

Le chitosan peut avantageusement présenter un taux de désacétylation d'au moins 50%, par exemple entre 55 et 90%, par exemple entre 70 et 80%.

Les particules comprenant un polymère cationique associé à au moins un ion métallique, peuvent avoir un diamètre pouvant aller de 0,01 µm à 1 mm, par exemple de 0,01 à 100 µm, par exemple de 0,1 à 10 µm. Le ou les ion(s) métallique(s) peu(ven)t être choisi(s) dans le groupe comprenant par exemple le cuivre, le zinc ou leur mélange.

Ledit polymère cationique et ledit ou lesdits ion(s) métallique(s) forme(nt) ensemble un complexe. Au sens de l'invention, un complexe désigne un édifice polyatomique constitué d'un ion métallique associé à au moins un groupement chimique capable de délocaliser une partie de sa densité électronique sur l'ion métallique, formant ainsi des liaisons de coordination avec celui-ci. Le groupement chimique peut être, par exemple, un groupement amine.

Pour 1 g de polymère cationique, la quantité d'ion(s) métallique(s) associé(s) peut être de 1 à 300 mg, par exemple de 10 à 200 mg.

Lorsque le polymère cationique est le chitosan, pour 1 g de chitosan, la quantité d'ion(s) métallique(s) associé(s) peut être de 20 à 200 mg, par exemple de 35 à 135 mg voire de 40 à 135 mg.

Selon la nature de l'ion métallique, et la nature des groupements chimiques présents dans les molécules indésirables, l'ion métallique peut former, de manière spécifique, un complexe avec lesdites molécules.

Selon un mode de réalisation de l'invention, lorsque les molécules indésirables sont des antibiotiques, les formes galéniques comprennent des particules pouvant contenir, en outre, au moins un agent capable d'inactiver lesdits antibiotiques. Les antibiotiques peuvent être, par exemple les quinolones, les aminoglucosides, les beta-lactames, les macrolides, les sulfamides, les antituberculeux, les tétracyclines. Ledit agent peut être une enzyme. A titre d'exemple, on peut citer les beta lactamases et/ou les érythromycine esterases. L'homme du métier saura choisir l'agent adapté en fonction de l'antibiotique à inactiver.

Comme déjà indiqué, les formes galéniques selon l'invention comprennent des particules capables d'adsorber, de manière spécifique, les molécules indésirables présentes dans le tube digestif. Afin d'augmenter davantage la stabilité des particules le long du tube digestif et afin d'éviter une adsorption prématurée de molécules, il est possible de renforcer les particules en les réticulant. Ainsi, dans un mode de réalisation particulier de l'invention les formes galéniques comprennent des particules sous forme réticulée. Les particules peuvent alors présenter un degré de réticulation allant de 0 à 100 %, par exemple de 1 à 99 %, par exemple de 10 à 80%. Lorsque le polymère cationique est un polysaccharide comportant des groupements amines, la réticulation peut avoir lieu avantageusement par lesdits groupements amines.

Le degré de réticulation peut alors être défini comme le pourcentage de groupements amines libres réticulés par l'agent réticulant, par rapport aux groupements amine initialement libres.

Le degré de réticulation des particules peut être déterminé par spectroscopie infrarouge, ou encore par titration [4] et [6].

Comme agent réticulant, on peut citer par exemple la glutaraldéhyde, le glycéraldéhyde, le formaldéhyde, l'épichlorohydrine, le tripolyphosphate.

Selon un autre mode de réalisation particulier de l'invention, les formes galéniques comprennent des particules telles que décrites précédemment, encapsulées dans des billes de pectine. Par billes de pectine, on entend des particules dont le diamètre moyen est compris entre 0,2 à 3 mm, par exemple de 0,5 à 1,7 mm, par exemple de 1 à 1,5 mm, obtenues par gélification ionique de gouttes d'une solution de pectine dans un bain d'une solution aqueuse contenant des cations multivalents, par exemple divalents ou trivalents [9]. Parmi ces ions multivalents on peut citer par exemple les ions calcium, les ions zinc, les ions aluminium, les ions fer ou les ions magnésium.

Au sens de l'invention, on entend par pectine aussi bien de la pectine méthylée, non méthylée, amidée ou non amidée. Plus particulièrement, les billes de pectine sont des billes de pectinate de zinc ou de calcium.

La pectine présente l'avantage d'être d'origine naturelle, dénuée de toxicité et pouvant spécifiquement être dégradée par les enzymes pectinolytiques présentes au niveau du colon. Une fois arrivées dans le colon, les billes se dégradent pour libérer les particules qui peuvent alors adsorber les molécules indésirables, comme par exemple les fluoroquinolones. L'encapsulation permet ainsi de renforcer davantage la stabilité des particules et de favoriser davantage une libération au niveau du colon.

Dans ce mode de réalisation, dans les formes galéniques, la concentration de pectine est de 5 à 95% en masse, par exemple de 25 à 50% en masse, par rapport à la masse totale de la forme galénique et celle de l'association polymère cationique-ion(s) métallique(s) est de 5 à 95% en masse, par exemple de 25 à 50% en masse par rapport à la masse totale de la forme galénique.

Toujours dans ce mode de réalisation, les billes de pectines peuvent être de forme sphérique et avoir un diamètre pouvant aller de 0,2 à 3 mm, par exemple de 0,5 à 1,7 mm, par exemple de 1 à 1,5 mm.

Dans un autre mode particulier de l'invention les particules, éventuellement encapsulées dans des billes de pectines, peuvent être enrobées avec :
- un polymère cationique choisi dans le groupe comprenant la polyéthylèneimine, la polylysine, la polyarginine, le DEAE dextran (ou diéthylaminoéthyl dextran) et le chitosan ;
- un polymère cellulosique choisi dans le groupe comprenant l'hydroxypropyle cellulose, l'hydroxyéthyle cellulose, l'hydroxyméthyle cellulose, l'hydroxypropyle méthyle cellulose, l'hydroxypropyle méthyle cellulose acétate succinate, l'hydroxypropyle méthyle cellulose phtalate, la méthyle cellulose, l'éthyle cellulose, l'acétate de cellulose, l'acétate de phtalate cellulose, l'acétate trimellitate cellulose et le carboxyméthyle cellulose de sodium ;
- un polymère d'acide acrylique, d'acide méthacrylique, de éthylacrylate, d'éthyleacrylate, de méthyle méthacrylate et/ou d'éthyle méthacrylate choisi dans le groupe comprenant les Eudragits® notamment Eudragit® NE, RL et RS, Eudragit® L30D-55 et L100-55, Eudragit® L100, Eudragit® S, et Eudragit® FS30D ;
- un polymère vinylique choisi dans le groupe comprenant le polyvinyle pyrrolidone, le vinyle acétate, le vinyle acétate phtalate, le vinyle acétate d'acide crotonique et éthylène-vinyle acétate.
Comme déjà indiqué, les formes galéniques de l'invention comprennent des particules éventuellement encapsulées et/ou enrobées capables d'adsorber, de manière spécifique, les molécules indésirables présentes dans le tube digestif.

L'enrobage permet de renforcer davantage la stabilité des particules éventuellement encapsulées, et de favoriser davantage une libération au niveau du tube digestif, avantageusement au niveau des intestins, en particulier au niveau de l'intestin grêle et/ou du colon.

Dans le cas de particules encapsulées dans des billes de pectine, par exemple des billes de pectinate de zinc, puis enrobées avec un polymère, par exemple un polymère de la famille des Eudragits, l'enrobage peut se dissoudre dans l'intestin grêle et la matrice de pectinate de zinc est ensuite dégradée par les enzymes pectinolytiques présentes au niveau du colon pour libérer les particules de chitosan-métal, lesquelles particules peuvent alors adsorber les molécules indésirables comme par exemple les antibiotiques.

La présente invention a également pour objet un procédé de préparation des formes galéniques telles que décrites précédemment, dans lequel :
*(a)* on dissout un polymère cationique qui est le chitosan dans une solution aqueuse d'un sel métallique ou d'un mélange des sels métalliques, dans des conditions pH < 7, avantageusement à un pH compris entre 1 et 6,8, encore plus avantageusement à un pH compris entre 1,2 et 6, de manière à ce que les ions métalliques dudit sel ou dudit mélange de sels s'associent audit polymère cationique de manière à former un complexe ;
*(b)* on soumet la solution obtenue en *(a)* à une atomisation-séchage, de manière à obtenir des particules dudit complexe ;
*(c)* on procède éventuellement à la réticulation des particules obtenues à l'étape *(b)* dans un solvant organique en présence d'un agent réticulant.

Selon un mode de réalisation, le polymère cationique est choisi parmi les polysaccharides cationiques et en particulier est le chitosan.

Dans l'étape *(a)*, le polymère cationique peut être dissous à une concentration comprise entre 0,2 et 10% (p/v) dans une solution aqueuse d'un sel métallique ou d'un mélange de sels métalliques à une concentration comprise entre 0,01M et 1M, par exemple 0,1M. Comme sel métallique on peut citer par exemple de cuivre, de zinc.

La dissolution s'effectue à un pH < 7, par exemple à un pH de 6,8 ; 6 ; 5 ; 2. Le temps de dissolution est fonction dudit pH : plus le pH est proche de 7, plus le temps de dissolution est long (environ quelques heures) et inversement, plus le pH est proche de 1, plus le temps de dissolution est court (environ 1 heure). La solution résultante peut être laissée sous agitation pendant une durée comprise entre 1h et 24h, par exemple 12h, à température ambiante (25°C). A l'issue de cette étape, on obtient une solution dans laquelle les ions métalliques sont associés au polymère cationique.

Dans l'étape *(b)*, on soumet la solution obtenue en (a) à une atomisation-séchage. Le séchage par atomisation (ou atomisation-séchage) est une technique d'élimination d'eau qui consiste à pulvériser un produit sous forme liquide, solution ou suspension, dans un courant de gaz chaud (air ou un gaz neutre). A l'issue de cette étape, les particules de polymère cationique obtenues sont sous forme d'une poudre avec notamment une coulabilité améliorée facilitant sa manutention et son dosage. L'atomisation-séchage étant un procédé bien connu, et l'homme du métier sera en mesure de déterminer les paramètres (par exemple la vitesse de séchage, la température du produit à sécher, la température d'entrée et de sortie de gaz etc.) pour une mise en oeuvre optimale.

Dans l'étape *(c)*, afin de procéder à la réticulation des particules, on met en suspension les particules obtenues à l'étape *(b)* dans un solvant organique et on y introduit un agent réticulant. Le solvant organique peut être choisi par exemple parmi le méthanol, l'éthanol, la pyridine, l'acétone, l'acide acétique, le DMSO, le dichlorométhane. Le milieu réactionnel peut contenir ou ne pas contenir de l'eau.

La suspension de particules peut être incubée sous agitation avec l'agent réticulant à température ambiante (25°C) pendant une durée comprise entre 10 minutes et 24h, par exemple 4h. Les particules, réticulées ou non, peuvent alors être filtrées et lavées dans un mélange d'eau et d'un solvant organique miscible à l'eau comme par exemple un mélange eau-éthanol, pour éliminer les ions métalliques ainsi que l'agent réticulant en excès. Les particules peuvent ensuite être séchées sous vide ou à l'étuve pendant une durée comprise entre 1h et 48h, par exemple 24h. La température de l'étuve peut être comprise entre 25 et 100 degrés celsius, par exemple 37°C.

Pour préparer les particules encapsulées, après l'étape *(b)* ou après l'étape *(c)*, les particules sont d'abord mises en suspension dans l'eau. La concentration des particules dans la suspension peut aller de 0,1 à 10% (p/v), par exemple 3%. Une solution de pectine, de concentration pouvant aller de 0,1 à 10% (p/v) par exemple 3% est alors mélangée avec la suspension de particules de chitosan-métal. Le mélange est alors formulé en faisant tomber des gouttes de ce mélange dans un bain contenant des cations multivalents, par exemple une solution d'acétate de zinc à 12% (p/v). La gélification ionique est obtenue en laissant les gouttes du mélange sous agitation dans la solution d'ions multivalents pendant un temps compris entre 1 minute et 24 heures, par exemple entre 5 minutes et 1 heure, par exemple entre 10 et 30 minutes. Les billes de pectine contenant des particules selon l'invention ainsi obtenues sont ensuite filtrées et rincées plusieurs fois dans de l'eau avantageusement ultra-pure (conductivité 18,2 MegaOhms.cm, système de purification Synergy de Millipore, France) par exemple entre 0 et 10 fois, par exemple entre 1 et 4 fois. Les billes de pectine contenant des particules selon l'invention ainsi obtenues, sont éventuellement séchées par exemple à l'étuve pendant une durée comprise entre 1 minute et 48 heures, par exemple entre 10 minutes et 24 heures par exemple 12 heures. La température de l'étuve peut être comprise entre 25 et 100 degrés celsius, par exemple 37°C.

Les billes de pectine contenant des particules selon l'invention ainsi obtenues peuvent aussi être lyophilisées.

Comme déjà indiqué, les particules éventuellement encapsulées peuvent, en outre, être enrobées avec un polymère tel que défini précédemment. A cet effet, l'enrobage peut s'effectuer avec une solution ou une suspension d'Eudragit® par exemple en utilisant un lit d'air fluidisé ou une turbine d'enrobage. Ces techniques sont bien connues de l'homme du métier dans le domaine de la galénique.

Un autre objet de la présente invention concerne les formes galéniques telles que décrites précédemment comme médicament. Ledit médicament peut comprendre, en outre, des composants bien connus de l'homme du métier dans le domaine pharmaceutique, comme des agents stabilisants, des agents émulsifiants, des agents de tonicité, des agents conservateurs, des colorants, des excipients, des liants, des lubrifiants.

Les formes galéniques selon l'invention peuvent être utilisées pour la prévention ou le traitement des effets indésirables liés à un déséquilibre de la flore intestinale et/ou colique suivant une antibiothérapie.

Un autre objet de l'invention consiste en l'utilisation d'une forme galénique selon l'invention pour la fabrication d'un médicament destiné à la prévention ou au traitement des effets indésirables liés à un déséquilibre de la flore intestinale et/ou colique suivant une antibiothérapie par exemple.

Les formes galéniques de l'invention peuvent être administrées sous toutes formes orales, notamment sous forme de gélules et de capsules. Les formes galéniques, peuvent être administrées avant, pendant ou après l'administration d'un antibiotique.

### Brève description des figures

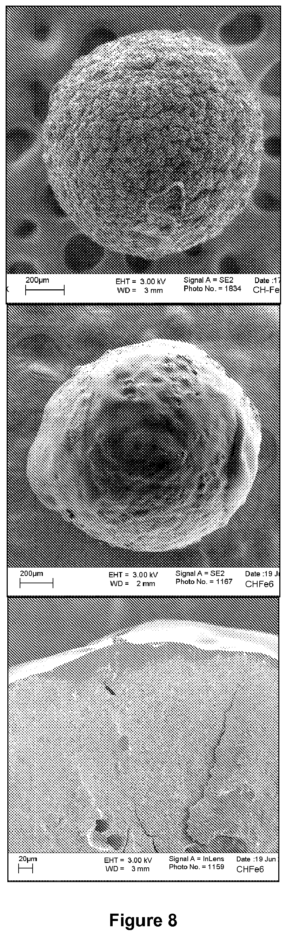

- La figure 1 représente un complexe chitosan-Fe(III).
- La figure 2 représente le schéma réactionnel de réticulation du chitosan par la glutaraldéhyde. L'étape A qui correspond à une étape de réticulation, a lieu sous agitation pendant 4 heures dans l'acétone.
- La figure 3 représente les images de microscopie électronique à balayage des particules de chitosan-Fe(III) (haut-gauche), de chitosan-Fe(II) (haut-droite), de chitosan-Cu (bas-gauche), de chitosan-Zn (bas-droite) après réticulation par la glutaraldéhyde et nettoyage.
- La figure 4 représente la cinétique d'adsorption de la ciprofloxacine à 400 µg/mL par les différentes particules réticulées en milieu colique simulé. Pour comparaison nous avons ajouté la cinétique obtenue avec le charbon actif dans les mêmes conditions.
   CH=particules de chitosan,
   CH-Cu=particules de chitosan-cuivre,
   CH-Zn=particules de chitosan-zinc,
   CH-Fe(II)=particules de chitosan-Fer(II),
   CH-Fe(III)=particules de chitosan-Fer(III),
   CH-Cu=particules de chitosan-cuivre.
   L'axe des ordonnées correspond la quantité de ciprofloxacine adsorbée (exprimée en µg) par les particules.

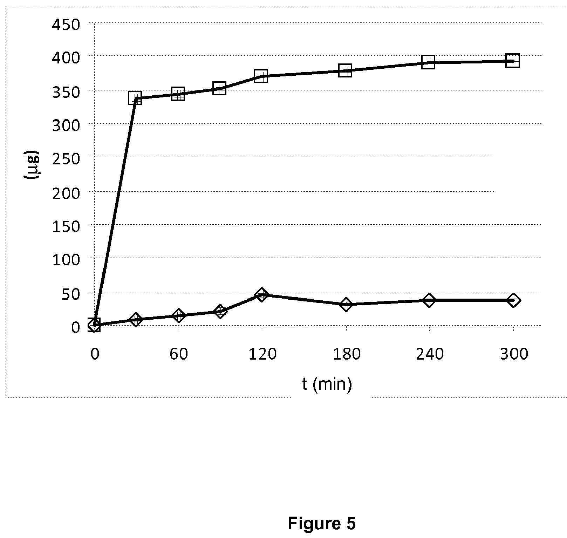

- La figure 5 représente les cinétiques d'adsorption de l'hydrocortisone par le charbon actif et des particules de chitosan-Fe(III). Les losanges représentent les particules de chitosan-Fe(III) et les carrés le charbon actif. L'axe des ordonnées correspond la quantité d'hydrocortisone adsorbée (exprimée en µg) par les particules.Ces résultats confirment la non spécificité des particules de chitosan-Fe(III) pour l'hydrocortisone.

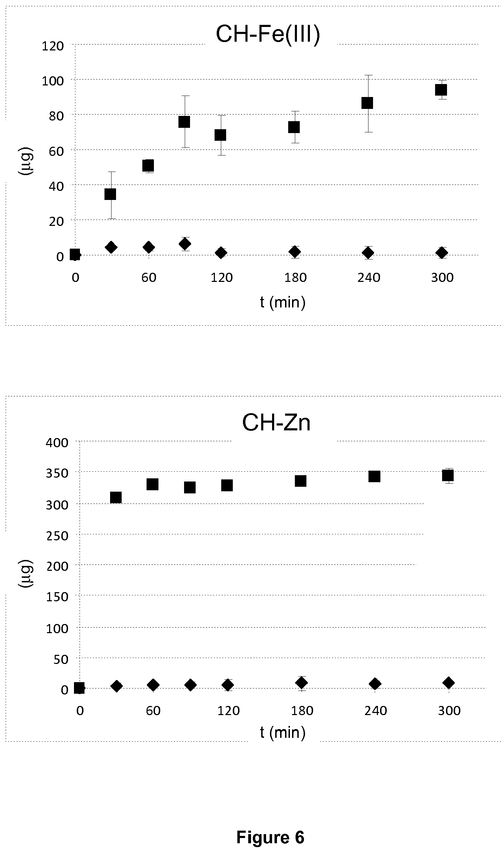

- La figure 6 représente les cinétiques d'adsorption de la ciprofloxacine en conditions de compétition avec la nimesulide, pour les particules de chitosan-Fe(III) et de chitosan-Zn. Les losanges représentent la nimesulide et les carrés la ciprofloxacine. L'axe des ordonnées correspond à la quantité de molécule adsorbée (exprimée en µg) par les particules. Ces résultats confirment la spécificité des particules de chitosan-Fe(III) et de chitosan-Zn pour la ciprofloxacine.

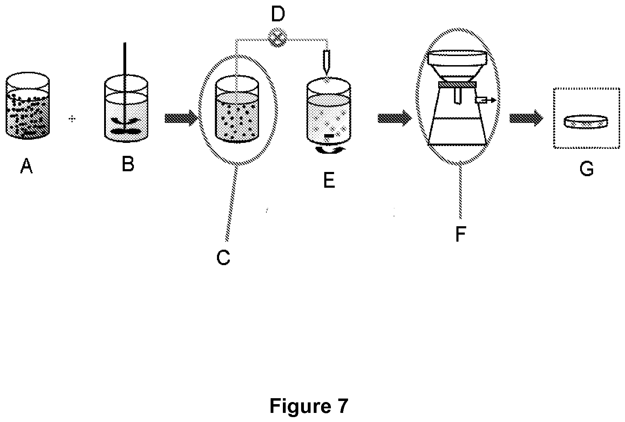

- La figure 7 représente le procédé de fabrication des billes de pectine contenant les particules selon l'exemple 6.
   A= suspension de particules
   B= solution de pectine
   C= mélange pectine/particules
   D= pousse seringue
   E= 50mL de solution d'acétate de zinc à 12%(p/v)
   F= lavage 1 minute dans 50mL d'eau
   G= séchage
- La figure 8 représente les images de microscopie électronique à balayage d'une bille de pectinate de zinc encapsulant les particules de chitosan-Fe(III) (en haut). L'image du milieu correspond à une bille enrobée avec de l'Eudragit® RS, et celle du bas à une coupe d'une bille enrobée : on distingue bien la couche d'Eudragit®.

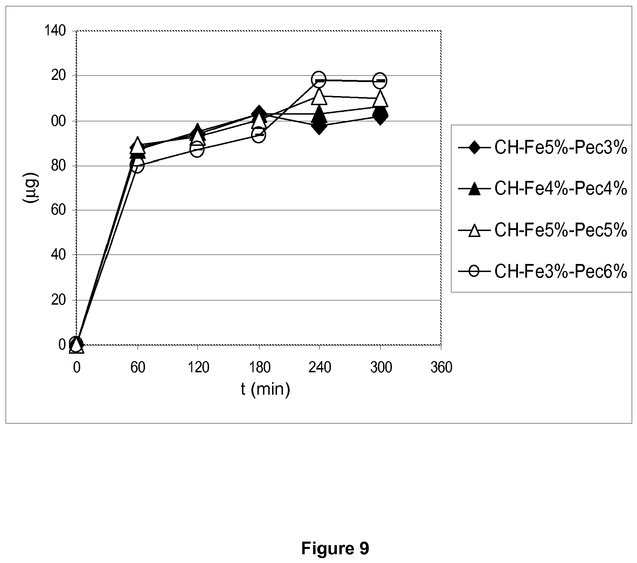

- La figure 9 représente les cinétiques d'adsorption des billes encapsulant les particules de chitosan-Fe(III) {ou CH-Fe(III)} dans le milieu intestinal simulé (SIM). Les billes ne sont pas enrobées par l'Eudragit®.

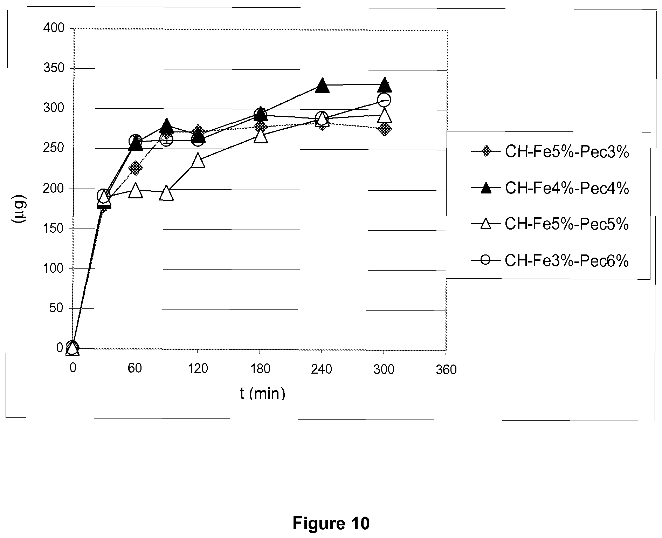

- La figure 10 représente les cinétiques d'adsorption des billes encapsulant les particules de chitosan-Fe(III) {ou CH-Fe(III)} dans le milieu colique simulé (SCM). Les billes ne sont pas enrobées par l'Eudragit® et ont été pré-incubées dans le SIM contenant la ciprofloxacine à 400 µg/mL. L'axe des ordonnées correspond à la quantité de ciprofloxacine adsorbée (exprimée en µg) par les particules encapsulées.

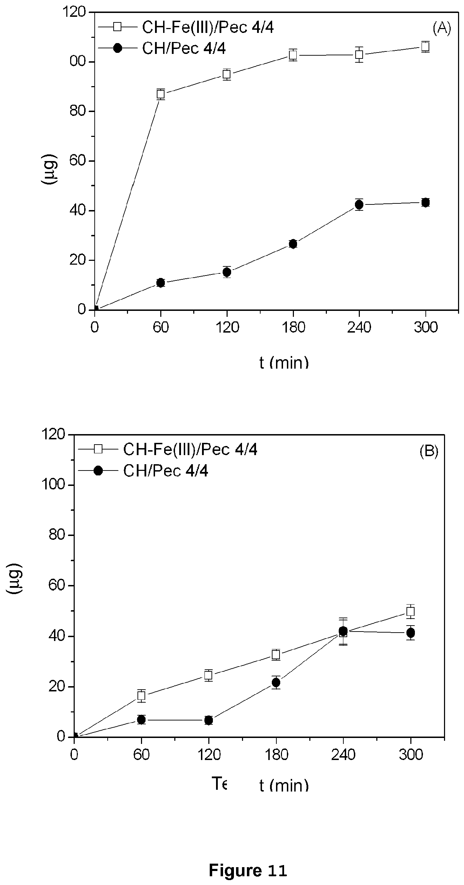

- La figure 11 représente les cinétiques d'adsorption de la ciprofloxacine à 400 µg/mL par des billes non enrobées (A) et enrobées par de l'Eudragit® (B) dans le SIM. L'axe des ordonnées correspond à la quantité de ciprofloxacine adsorbée (exprimée en µg) par les particules encapsulées.

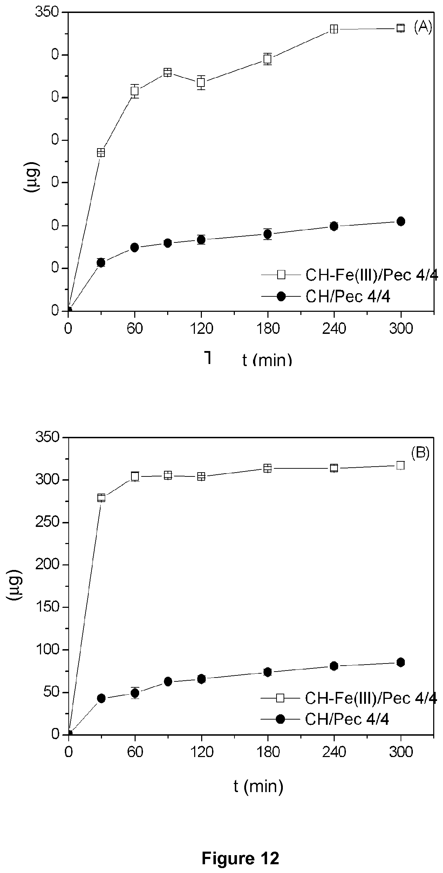

- La figure 12 représente les cinétiques d'adsorption de la ciprofloxacine à 400 µg/mL par des billes non enrobées (A) et enrobées par de l'Eudragit® (B) dans le SCM, après préincubation dans le SIM. L'axe des ordonnées correspond à la quantité de ciprofloxacine adsorbée (exprimée en µg) par les particules encapsulées.

### EXEMPLES

### Exemple 1 : Complexation des ions métalliques par le chitosan

Le Chitosan (de bas poids moléculaire - low molecular weight en anglais), l'acétate de zinc dihydrate et la ciprofloxacine proviennent de chez Fluka (Suisse). La glutaraldehyde, le sulfate de cuivre(II) et l'acétate de sodium proviennent de chez Prolabo (Paris-France). Le chlorure de fer (II) vient de chez Acros Organic (Geel, Belgique). L'acide hydroxyéthylpipérazine-éthanesulfonique (HEPES), le 1-(2-pyridylazo)-2-naphthol (PAN), le tampon ammonia buffer (pH 10) pour complexométrie, l'hydrochlorure d'hydroxylamine, la 1,10-phenantroline et le nitrate de fer(III) ont été achetés chez Sigma-Aldrich (France). Les solvants de grade HPLC proviennent de chez by Carlo Erba (Italie).

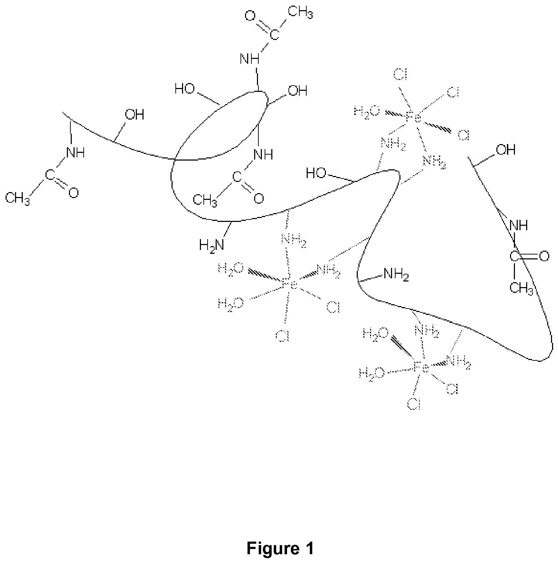

Le chitosan a été complexé avec les ions métalliques (cuivre, fer (II), fer (III) et zinc) en dissolvant 1g de chitosan dans 100mL d'une solution aqueuse d'ions métalliques à 0,1M (CuSO₄, FeCl₂, Fe(NO₃)₃ et Zn(CH₃COO)₂). Les solutions ont alors été agitées pendant 12 heures à température ambiante (25°C). La complexation a eu lieu via les groupements amines « libres » du chitosan selon la figure 1. Les ions métalliques sont ajoutés en léger excès par rapport au nombre de groupements amines « libres » du chitosan.

### Exemple 2 : Préparation des formes galéniques

Les complexes chitosan-ion métallique (cuivre, fer (II), fer (III) et zinc) préparés dans l'exemple 1, sont soumis à une atomisation pour former les particules. 200mL de la solution de complexe sont atomisés au moyen d'un appareil spray-dryer B191 (Büchi, France). La buse possède un diamètre interne de 0,7mm et fonctionne avec de l'air comprimé à un débit de 600L/h. La température d'entrée de l'appareil est fixée à 150°C. La température de sortie est comprise entre 75 et 100°C. Le débit de la pompe amenant la solution à atomiser est fixé à 5mL/min. Les particules sont récupérées sont forme de poudre dans le cyclone de l'appareil.

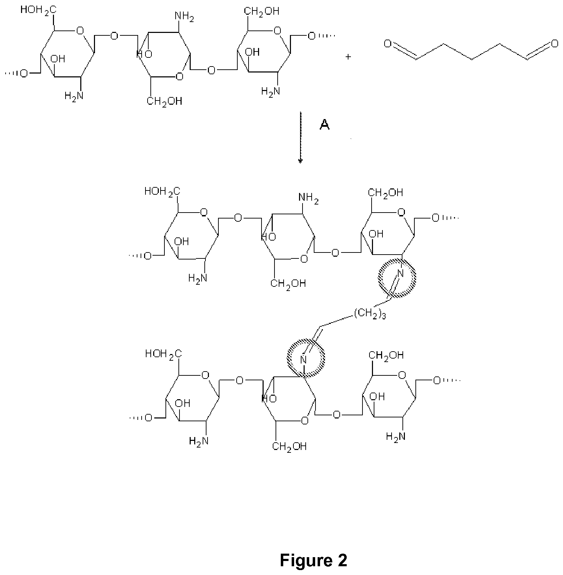

Les particules sont ensuite suspendues dans l'acétone avec de la glutaraldéhyde (rapport molaire chitosan glutaraldéhyde 1:1) pendant 4 heures sous agitation magnétique pour obtenir la réticulation (figure 2). Les particules sont ensuite rincées abondamment avec un mélange éthanol/eau (2/1, v/v) pour éliminer la glutaraldéhyde et les ions métalliques résiduels. Les particules nettoyées ont finalement été séchées sous vide pendant 24h.

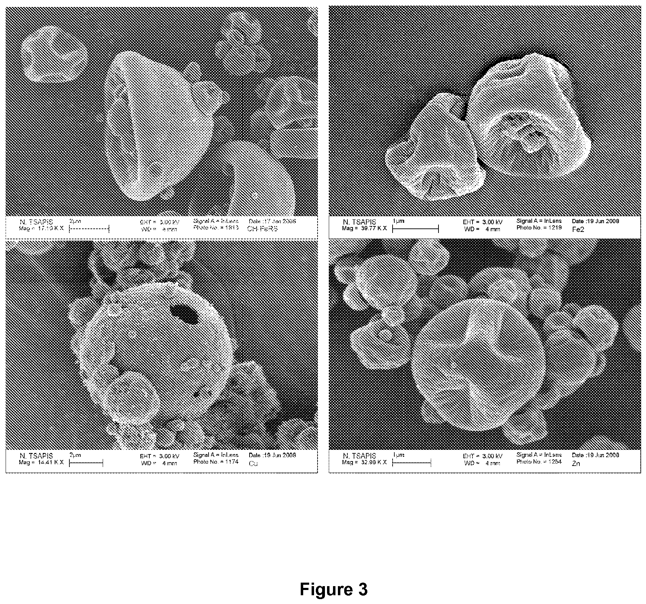

Les observations de microscopie électronique à balayage ont été réalisées avec un microscope LEO 1530 LEO 1530 (LEO Electron Microscopy Inc, Thornwood, NY) à une tension d'accélération de 3kV et un courant de 0,5mA. Les particules ont été déposées sur une bande adhésive conductrice (Euromedex, France) avant d'être métallisées avec 2nm d'une couche d'alliage platine-palladium avec un appareil Cressington 208HR (USA) .La microscopie électronique à balayage montre que les particules possèdent une surface relativement lisse (figure 3).

Dans les particules, la quantité d'ions métalliques associées au chitosan a été dosée par colorimétrie : 131mg Fe(III)/1g chitosan ; 45mg Fe(II)/1g chitosan et 87 mg Zn/1g chitosan. Plus particulièrement, la quantité de fer (Fe(II) ou Fe(III)) a été dosée en utilisant la 1,10-phenantroline par spectrophotométrie : Les particules de CH-Fe(II) ou de CH-Fe(III) ont d'abord été soumises à une hydrolyse en milieu acide (100mg de particules dans 5mL d'un mélange HCl : HNO₃ 1:1). 1 mL de la solution a ensuite été placé dans une fiole jaugée de 10mL dans laquelle ont été ajoutés 2mL d'une solution d'hydroxylamine HCl (1,4M), 5mL de tampon acétate (2M) et 2mL de 1,10 phenantroline (5mM). Après 20 minutes un complexe rouge-orangé se forme dont on mesure l'absorbance à 510nm par spectrophotométrie (Shimadzu, France) contre un blanc ne contenant pas le fer. L'absorbance à 510nm est proportionnelle à la quantité de fer. Plus particulièrement, la quantité de zinc a été dosée en utilisant une méthode décrite par M. Khoder et al. [7] et M. Arvand et al. [8]. (Les particules de CH-Zn ont d'abord été soumises à une hydrolyse en milieu acide (100mg de particules dans 5mL d'un mélange HCl : HNO₃ 1 :1). 1 mL de la solution a ensuite été placé dans une fiole jaugée de 10mL dans laquelle ont été ajoutés 1mL d'un tampon ammoniac (1M, pH=10) et 3mL de 1-(2-pyridylazo)-2-naphthol (PAN) (5×10⁻⁴M) dans l'éthanol. Le volume est ensuite complété à 10mL avec de l'éthanol. La fiole est ensuite agitée et placée dans un bain thermostaté à 40°C pendant 10minutes pour obtenir la formation du complexe PAN-Zn. L'absorbance à 550nm est ensuite mesurée avec un spectrophotomètre Shimadzu (France) contre un blanc, elle est proportionnelle à la quantité de zinc.

### Exemple 3 : Etude de l'adsorption de molécule indésirable

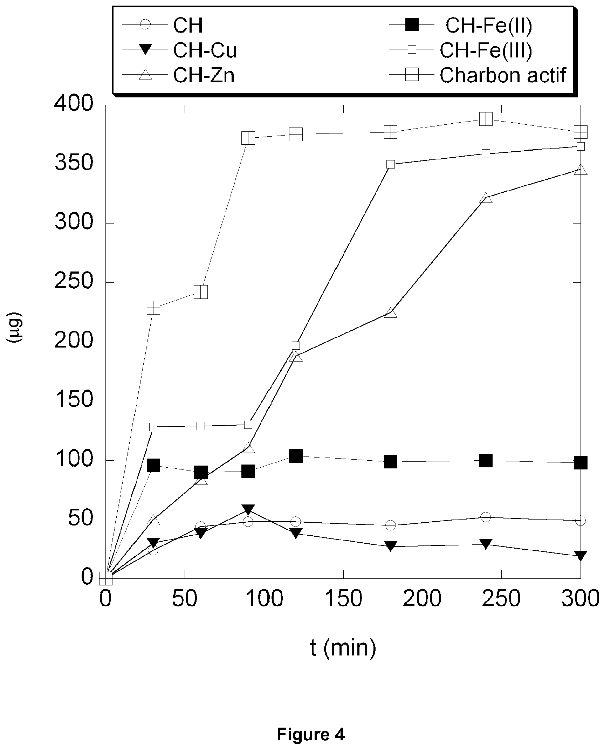

Les particules (1mg/mL) ont été incubées à 37°C dans un milieu colique simulé constitué de tampon HEPES (10mM) et de NaCl (145mM) contenant 5200PG/mL d'enzymes pectinolytiques (Pectinex, Sigma-Aldrich) contenant la ciprofloxacine à une concentration de 400 µg/mL, le pH a été ajusté à 5,5 avec du NaOH (1N). Les cinétiques d'adsorption de la ciprofloxacine par les particules réticulés de chitosan (contrôle), de chitosan-Cu, de chitosan-Fe(II), de chitosan-Fe(III) et de chitosan-Zn ont été évaluées pendant 5 heures (figure 4).

Pour les particules de chitosan, de chitosan-Cu, de chitosan-Fe(II), le plateau d'adsorption est atteint rapidement (30 minutes) et se situe à 50, 25 et 100 µg respectivement.

Le plateau d'adsorption est atteint moins rapidement pour le chitosan-Zn et le chitosan-Fe(III) : 5 heures et 3 heures respectivement. La quantité de ciprofloxacine adsorbée par le chitosan-Zn et le chitosan-Fe(III) est de 350 µg, soit presqu'autant que le charbon actif (400 µg) considéré comme un bon adsorbant mais non spécifique. Les particules de chitosan-Zn et de chitosan-Fe(III) peuvent donc être considérées comme des adsorbants particulièrement efficaces vis-à-vis de la ciprofloxacine.

### Exemple 4 : Etude de la spécificité d'adsorption

L'adsorption spécifique des fluoroquinolones par les particules de l'invention est réalisée. Des particules chitosan-Fe(III) (1 mg/mL) sont incubées à 37°C dans un milieu colique simulé identique à celui décrit dans l'exemple 3 mais contenant de l'hydrocortisone, à la même concentration que la ciprofloxacine dans l'exemple 3, soit 400 µg/mL.

Alors que la ciprofloxacine est adsorbée à hauteur de 350 µg environ par les particules de chitosan-Fe(III), l'hydrocortisone est faiblement adsorbée par les particules de chitosan-Fe(III): 50 µg < (figure 5). En revanche, le charbon actif (1 mg/mL) entraîne une forte adsorption de l'hydrocortisone. Ces résultats confirment la spécificité des particules selon l'invention pour les fluoroquinolones.

### Exemple 5 : Compétition entre la ciprofloxacine et la nimesulide

L'adsorption spécifique d'un fluoroquinolone, la ciprofloxacine, par les particules de l'invention est réalisée en conditions de compétition avec la nimesulide. Des particules chitosan-Fe(III) et chitosan-Zn (1 mg/mL) sont incubées à 37°C dans un milieu colique simulé identique à celui décrit dans l'exemple 3 contenant de la nimesulide (100 µg/mL) et de la ciprofloxacine (400 µg/mL). Pour cet exemple le pH a été ajusté à 6.

Alors que la ciprofloxacine est adsorbée à hauteur de 350 µg environ par les particules de chitosan-Zn et à hauteur de 100 µg pour les particules de chitosan-Fe(III), la nimesulide est faiblement adsorbée par les deux types de particules: 10 µg < (figure 6). Ces résultats confirment la spécificité des particules selon l'invention pour les fluoroquinolones.

### Exemple 6 : Encapsulation des particules

La pectine, polysaccharide naturel (extrait à partir d'agrumes, famille des citrus) est employée sous sa forme amidée (19 à 23%) et faiblement méthylée (22 à 28 %). Nous l'obtenons auprès de Cargill (Belgique) sous le nom de marque Unipectine OG175C.

### Préparation de billes ne contenant pas de particules

Des billes ne contenant pas de particules sont préparées en introduisant goutte-à-goutte, à travers une buse d'un diamètre interne de 0,8 mm, une solution aqueuse de pectine à une concentration de 1 à 10 % (p/v) dans une solution d'un sel de zinc tel que l'acétate de zinc (0,5 à 12% (p/v)) ou un sel de calcium tel que le chlorure de calcium (0,5% à 12% (p/v)), pour former des billes de pectinate de zinc ou de pectinate de calcium. Les billes obtenues sont récupérées après un temps de résidence de 30 minutes dans le bain des contre-ions. Les billes ainsi obtenues sont ensuite récupérées par filtration, rincées 3 fois 1 minute à l'eau ultra-pure (conductivité 18,2MegaOhms.cm, système de purification Synergy de Millipore, France) afin d'éliminer les ions divalents qui ne participent pas au réseau, et séchées à l'étuve à 37°C pendant 12 heures.

### Préparation de billes contenant des particules

La préparation de billes contenant des particules de chitosan ou de chitosan-metal est légèrement différente (figure 7). Les particules sont suspendues dans l'eau à une concentration de 4% (p/v). Une solution de pectine à 4% (p/v) est ensuite ajoutée à la suspension à volume égal et l'ensemble est mélangé au moyen d'un agitateur du type vortex (commercialisé par exemple par la société VWR) ou avec une pâle d'agitation, par exemple hélicoïdale. Les billes contenant les particules sont alors formées en introduisant goutte-à-goutte, à travers une buse d'un diamètre interne de 0,8 mm, le mélange pectine/particules dans une solution d'un sel de zinc tel que l'acétate de zinc (0,5 à 12% (p/v)) ou un sel de calcium tel que le chlorure de calcium (0,5% à 12% (p/v)), pour former des billes de pectinate de zinc ou de pectinate de calcium (Figure 7). Les billes obtenues sont récupérées après un temps de résidence de 30 minutes dans le bain des contre-ions. Les billes ainsi obtenues sont ensuite récupérées par filtration, rincées 3 fois 1 minute à l'eau ultra-pure (conductivité 18,2 MegaOhms.cm, système de purification Synergy de Millipore, France) afin d'éliminer les ions divalents qui ne participent pas au réseau, et séchées à l'étuve à 37°C pendant 12 heures. Les interactions électrostatiques entre les groupements carboxylate de la pectine et les groupements amine du chitosan entraînent une gélification du mélange pectine/particules. Le temps de gélification dépend de la concentration initiale des deux composants.

### Billes de pectine contenant du chitosan-Fe(III)

Du fait des problèmes de gélification, pour une concentration de pectine finale dans le mélange de 3% (p/v), il est possible de formuler des billes sphériques contenant jusqu'à 1,5% (p/v) de chitosan-Fe(III). Pour une concentration de pectine finale dans le mélange de 2,5% (p/v), il est possible de formuler des billes sphériques contenant jusqu'à 2,5% (p/v) de chitosan-Fe(III). Pour une concentration de pectine finale dans le mélange de 2% (p/v), il est possible de formuler des billes sphériques contenant jusqu'à 2% (p/v) de chitosan-Fe (III). Pour une concentration de pectine finale dans le mélange de 1,5% (p/v), il est possible de formuler des billes sphériques contenant jusqu'à 2,5% (p/v) de chitosan-Fe (III). Au-delà de ces concentrations de particules la suspension peut devenir trop visqueuse et des sortes de vermicelles peuvent être obtenues.

### Billes de pectine contenant du chitosan-Zn

Pour une concentration finale de pectine dans le mélange de 1,5% (p/v), il est possible de formuler des billes sphériques contenant jusqu'à 0,5% de chitosan-Zn. Au-delà de ces concentrations de particules, la suspension peut devenir trop visqueuse et des sortes de vermicelles peuvent être obtenues. Avec le chitosan-Zn, les ions de Zn participent à la gélification de la pectine.

L'observation des billes en microscopies optique et électronique montre des particules de formes sphériques, de tailles assez homogènes entre 1 à 1,3 mm et d'une masse qui varie de 1 à 2 mg. Une dispersion uniforme des particules de chitosan-métal ainsi qu'une surface rugueuse est observées en microscopie électronique à balayage

### Exemple 7 : Etude de l'adsorption de molécule indésirable

Les billes de pectinate de zinc encapsulant des particules de chitosan-métal ont été incubées dans le milieu intestinal simulé (SIM) contenant 400 µg/mL de ciprofloxacine pendant 5 heures. Le milieu intestinal simulé est une solution aqueuse de tampon HEPES (30mM) contenant 1% de pancréatine (p/v), le pH est ajusté à 6,8 avec du NaOH 0,2M. Les billes gonflent mais restent intactes au terme de ces 5 heures d'incubation. Nous avons tout de même observé qu'une partie de la ciprofloxacine dans le SIM était adsorbée : environ 100 à 120 µg au bout de 5 heures (figure 9).

Après pré-incubation dans le SIM contenant la ciprofloxacine, les billes conservent leur pouvoir adsorbant puisque jusqu'à 330 µg de ciprofloxacine dans le milieu colique simulé (SCM, décrit ci-dessus) est adsorbée (figure 10).

Si les quantités de ciprofloxacine dans le SIM et dans le SCM adsorbées sont additionnées, on peut trouver que l'équivalent en bille de 1 mg de particules de chitosan-Fe(III) réussit à adsorber environ 350 µg de ciprofloxacine, soit ce qui avait été obtenu pour les particules non encapsulées. L'encapsulation des particules dans les billes de pectinate de zinc ne modifie donc pas leur pouvoir adsorbant.

### Exemple 8 : Enrobage des particules encapsulées

Afin de limiter l'adsorption dans le milieu intestinal simulé (SIM), nous avons enrobé les billes sèches avec 5 % (p/p) d'Eudragit® RS. Pour cela, 5g d'Eudragit® RS et 1g de PEG 300 (Sigma-Aldrich) sont dissouts dans 300mL d'un mélange acétone/éthanol (2/1, v/v). La solution est ensuite atomisée au moyen d'un pulvérisateur manuel sur les billes sèches maintenues en rotation à 20 tours/minute dans une miniturbine chauffée à 37°C avec un pistolet à air chaud (Steinel, Radiospares, France). La prise en poids de 5 à 10% a été vérifiée par pesée. L'homogénéité de l'enrobage est vérifiée par microscopie électronique à balayage (figure 8).

L'incubation des billes enrobées dans le SIM décrit ci-dessus à 400 µg/mL de ciprofloxacine montre que l'Eudragit® RS permet de limiter l'adsorption de la ciprofloxacine : 25 - 30 µg au lieu de 100 à 120 µg pour les billes non enrobées (figure 11). Cette couche d'Eudragit ne modifie pas le pouvoir adsorbant des billes : après préincubation dans le SIM, les billes enrobées peuvent encore adsorber 300 µg de ciprofloxacine (figure 12).

### Listes des références

**[1]** Bartlett J.G., New England Journal of Medicine, 346, p.334-339, 2002.
**[2]** Holmberg S.D. et al., New England Journal of Medicine, 311, 617, 1984.
**[3]** Bartlett J.G., Clostridium difficile infection: pathophysiology and diagnosis. Seminar in Gastrointestinal Disease 8, 12, 1997.
**[4]** Broussignac, P. Chim. Ind. Gén. Chim., no. 99, p. 1241-1246, 1968.
**[5]** Médicaments et environnement - rapport de l'academie nationale de pharmacie, p. 23/103 - 25/103, septembre 2008
**[6]** K.C. Gupta et al., Carbohydrate Polymers, 66, p. 43-45, 2006 ; K.C. Gupta et al., Carbohydrate Research, 342, p. 2244-2252, 2007.
**[7]** M. Khoder et al., International Journal of Pharmaceutics, 379, p. 251-259,2009.
**[8]** M. Arvand et al., J. Anal. Chem., 62, p. 342-347, 2007.
**[9]** I. El-Gibaly, International Journal of Pharmaceutics, 232, p. 199-211, 2002.

## Revendications

1. Forme galénique comprenant des particules capables d'adsorber, de manière spécifique, des molécules indésirables présentes dans le tube digestif, lesdites particules comprenant (i) un polymère cationique qui est le chitosan, associé à (ii) au moins un ion métallique choisi dans le groupe comprenant, le cuivre, le zinc ou leur mélange , ledit polymère cationique et ledit au moins un ion métallique formant un complexe.

2. Forme galénique selon la revendication1 , dans laquelle le chitosan présente un taux de désacétylation d'au moins 50%.

3. Forme galénique selon l'une quelconque des revendications 1 à 2, dans laquelle pour 1 g de polymère cationique, la quantité d'ion(s) métallique(s) associé(s) est de 1 à 300 mg, ou dans laquelle pour 1 g de polymère cationique, la quantité d'ion(s) métallique(s) associé(s) est de 20 à 200 mg.

4. Forme galénique selon l'une quelconque des revendications 1 à 3, dans laquelle les particules ont un diamètre allant de 0,01 µm à 1 mm.

5. Forme galénique selon l'une quelconque des revendications 1 à 4, dans laquelle les particules sont capables d'adsorber, de manière spécifique, les molécules indésirables choisies dans le groupe comprenant :
• les substances toxiques domestiques ;
• les résidus médicamenteux ou les métabolites des médicaments qui sont ingérés de manière volontaire et/ou accidentelle ;
• les résidus médicamenteux ou les métabolites des médicaments les plus employés en médecine humaine ou vétérinaire susceptibles de se retrouver dans les eaux ;
• les antibiotiques résiduels, ou dans laquelle les particules sont capables d'adsorber, de manière spécifique, les antibiotiques résiduels choisis dans le groupe comprenant les quinolones, les aminoglucosides, les beta-lactames, les macrolides, les sulfamides, les antituberculeux et les tétracyclines.

6. Forme galénique selon la revendication 5, dans laquelle les molécules indésirables sont des antibiotiques résiduels, éventuellement choisis parmi les quinolones, les aminoglucosides, les beta-lactames, les macrolides, les sulfamides, les antituberculeux et les tétracyclines, et les particules contiennent, en outre, au moins un agent capable d'inactiver lesdits antibiotiques, en particulier une enzyme.

7. Forme galénique selon l'une quelconque des revendications 1 à 6, dans laquelle les particules présentent un degré de réticulation allant de 0 à 100%.

8. Forme galénique selon l'une quelconque des revendications 1 à 7, dans laquelle les particules sont encapsulées dans des billes de pectine, ou dans laquelle les particules sont encapsulées dans des billes de pectinate de zinc ou de pectinate de calcium.

9. Forme galénique selon la revendication 8, dans laquelle la concentration de pectine est de 5 à 95% en masse par rapport à la masse totale de la forme galénique et celle de l'association polymère cationique-ion(s) métallique(s) est de 5 à 95% en masse par rapport à la masse totale de la forme galénique.

10. Forme galénique selon l'une quelconque des revendications 8 à 9, dans laquelle les billes de pectines sont de forme sphérique et de diamètre allant de 0,2 à 3 mm.

11. Forme galénique selon l'une quelconque des revendications 1 à 10, dans laquelle les particules, éventuellement encapsulées dans des billes de pectines, sont enrobées avec :
- un polymère cationique choisi dans le groupe comprenant les polyéthylèneimines, les polylysines, les polyarginines, le DEAE dextran et le chitosan ;
- un polymère cellulosique choisi dans le groupe comprenant l'hydroxypropyle cellulose, l'hydroxyéthyle cellulose, l'hydroxyméthyle cellulose, l'hydroxypropyle méthyle cellulose, l'hydroxypropyle méthyle cellulose acétate succinate, l'hydroxypropyle méthyle cellulose phtalate, la méthyle cellulose, l'éthyle cellulose, l'acétate de cellulose, l'acétate de phtalate cellulose, l'acétate trimellitate cellulose et le carboxyméthyle cellulose de sodium ;
- un polymère d'acide acrylique, d'acide méthacrylique, de éthylacrylate, d'éthyleacrylate, de méthyle méthacrylate et/ou d'éthyle méthacrylate choisi dans le groupe comprenant les Eudragits® notamment Eudragit® NE, RL et RS, Eudragit® L30D-55 et L100-55, Eudragit® L100, Eudragit® S, et Eudragit® FS30D ;
- un polymère vinylique choisi dans le groupe comprenant le polyvinyle pyrrolidone, le vinyle acétate, le vinyle acétate phtalate, le vinyle acétate d'acide crotonique et l'éthylène-vinyle acétate.

12. Procédé de préparation d'une forme galénique selon l'une quelconque des revendications 1 à 11, dans lequel :
*(a)* on dissout un polymère cationique qui est le chitosan dans une solution aqueuse d'un sel métallique ou d'un mélange des sels métalliques, dans des conditions pH < 7, avantageusement à un pH compris entre 1 et 6,8, encore plus avantageusement à un pH compris entre 1,2 et 6, de manière à ce que les ions métalliques dudit sel ou dudit mélange de sels s'associent audit polymère cationique de manière à former un complexe ;
*(b)* on soumet la solution obtenue en (*a*) à une atomisation-séchage, de manière à obtenir des particules dudit complexe;
*(c)* on procède éventuellement à la réticulation des particules obtenues à l'étape *(b)* dans un solvant organique en présence d'un agent réticulant.

13. Forme galénique selon l'une quelconque des revendications 1 à 11 comme médicament.

14. Forme galénique selon l'une des revendications 1 à 11 pour son utilisation comme médicament afin de traiter des effets indésirables liés à un déséquilibre de la flore intestinale et/ou une colique suivant une antibiothérapie.

15. Forme galénique selon la revendication 14 dans laquelle le médicament est administré avant, pendant ou après l'administration d'un antibiotique.

## Patentansprüche

1. Galenische Form umfassend Partikel, die im Verdauungstrakt vorhandene unerwünschte Moleküle spezifisch absorbieren können, wobei die Partikel folgende umfassend : (i) ein kationisches Polymer, das Chitosan ist, das mit (ii) mindestens einem Metallion verbunden ist, das aus der Gruppe ausgewählt ist, die Kupfer, Zink oder eine Mischung davon umfasst, wobei das kationische Polymer und das mindestens eine Metallion einen Komplex bilden.

2. Galenische Form nach Anspruch 1, wobei das Chitosan einen Deacetylierungsgrad von mindestens 50 % aufweist.

3. Galenische Form nach einem der Ansprüche 1 bis 2, wobei für 1g des kationischen Polymers die Menge des/der verbundenen Metallions/-ionen 1 bis 300 mg beträgt oder wobei für 1g des kationischen Polymers die Menge des/der verbundenen Metallions/-ionen 20 bis 200 mg beträgt.

4. Galenische Form nach einem der Ansprüche 1 bis 3, wobei die Partikel einen Durchmesser von 0,01 µm bis 1 mm aufweisen.

5. Galenische Form nach einem der Ansprüche 1 bis 4, wobei die Partikel unerwünschte Moleküle spezifisch absorbieren können, die aus der Gruppe ausgewählt sind, die Folgende umfasst :
• Toxische Substanzen im Haushalt ;
• Medikamentöse Rückstände oder Metaboliten von Medikamenten, die vorsätzlich und/oder unbeabsichtigt eingenommen werden ;
• Medikamentöse Rückstände oder Metaboliten von den in der Human- und Tiermedizin gebräuchlichsten Medikamenten, die sich im Wasser wiederfinden können ;
• Antibiotikarückstände, wobei die Partikel fähig sind, spezifisch Antibiotikarückstände zu absorbieren, die aus der Gruppe umfassend Chinolone, Aminoglycoside, Betalactame, Makrolide, Sulfonamide, Tuberkulosbekämpfungsmittel und Tetracycline ausgewählt sind.

6. Galenische Form nach Anspruch 5, wobei die unerwünschten Moleküle Antibiotikarückstände sind, die gegebenenfalls unter folgenden ausgewählt sind : Chinolone, Aminoglycoside, Betalactame, Makrolide, Sulfonamide, Tuberkulosbekämpfungsmittel und Tetracycline und die Partikel ferner mindestens ein Mittel, insbesondere eine Enzym, umfassen, das die Antibiotika inaktivieren kann.

7. Galenische Form nach einem der Ansprüche 1 bis 6, wobei die Partikel einen Vernetzungsgrad von 0 bis 100 % aufweisen.

8. Galenische Form nach einem der Ansprüche 1 bis 7, wobei die Partikel in Pektinkügelchen eingekapselt sind oder wobei die Partikel in Zinkpektinat- oder Kalziumpektinatkügelchen eingekapselt sind.

9. Galenische Form nach Anspruch 8, wobei die Pektinkonzentration 5 bis 95 Massen-% beträgt, bezogen auf die Gesamtmasse der galenischen Form und der kationischen Polymer-Metallion(en)-Verbindung 5 bis 95 Massen-% beträgt, bezogen auf die Gesamtmasse der der galenischen Form.

10. Galenische Form nach einem der Ansprüche 8 bis 9, wobei die Pektinkügelchen eine sphärische Form und einen Durchmesser von 0,2 bis 3 mm haben.

11. Galenische Form nach einem der Ansprüche 1 bis 10, wobei die gegebenenfalls in Pektinkügelchen verkapselten Partikel, mit Folgenden beschichtet sind:
- einem kationisches Polymer ausgewählt aus der Gruppe, die Polyethylenimin, Polylysin, Polyarginin, DEAE-Dextran und Chitosan umfasst ;
- einem Cellulosepolymer, ausgewählt aus der Gruppe, die Hydroxypropylcellulose, Hydroxyethylcellulose, Hydroxymethylcellulose, Hydroxypropylmethylcellulose, Hydroxypropylmethylcelluloseacetatsuccinat, Hydroxypropylmethylcellulosephthalat, Methylcellulose, Ethylcellulose, Celluloseacetat, Cellulosephthalatacetat, Celluloseacetattrimellitat und Natriumcarboxymethylcellulose umfasst;
- einem Polymer der Acrylsäure, der Methacrylsäure, des Ethylacrylats, des Ethyleacrylats, des Methylmethacrylats und / oder des Ethylmethacrylats ausgewählt aus der Gruppe, die Eudragiten® insbesondere Eudragit® NE, RL und RS, Eudragit® L30D -55 und L100-55, Eudragit® L100, Eudragit® S und Eudragit® FS30D umfasst;
- einem Vinylpolymer, ausgewählt aus der Gruppe, die Polyvinylpyrrolidon, Vinylactetat, Vinylacetatphthalat, Vinylacetat-Crotonsäure und Ethylenvinylacetat umfasst.

12. Verfahren zur Herstellung einer galenischen Form nach einem der Ansprüche 1 bis 11, wobei :
(a) ein kationisches Polymer, das Chitosan ist, in einer wässrigen Lösung eines Metallsalzes oder einer Mischung von Metallsalzen bei einem pH-Wert <7, vorteilsweise bei einem pH-Wert zwischen 1 und 6,8, und noch vorteilhafter bei einem pH-Wert von 1,2 bis 6 aufgelöst wird, so daß die Metallionen des Salzes oder Salgemisches sich mit dem kationischen Polymer verbinden um einen Komplex zu bilden;
(b) die in (a) erhaltene Lösung einer Sprühtrocknung unterzogen wird, um die Partikel des Komplexes zu erhalten ;
(c) gegebenenfalls die Vernetzung der in Schritt (b) erhaltenen Partikel in einem organischen Lösungsmittel in Gegenwart eines Vernetzungsmittels durchgeführt wird..

13. Galenische Form nach einem der Ansprüche 1 bis 11 als Medikament.

14. Galenische Form nach einem der Ansprüche 1 bis 11 zur Verwendung als Medikament zur Behandlung von Nebenwirkungen im Zusammenhang mit einem Ungleichgewicht in der Darmflora und / oder einer Kolik infolge einer Antibiotikatherapie.

15. Verwendung nach Anspruch 14, wobei das Medikament bevor, während oder nach der Verabreichung eines Antibiotikums verabreicht wird.

## Claims

1. A galenic form comprising particles capable of specifically adsorbing undesirable molecules present in the digestive tract, said particles comprising (i) a cationic polymer chosen that is chitosan, associated with (ii) at least one metal ion chosen from copper, zinc or a mixture thereof, said cationic polymer and said at least one metal ion forming a complex.

2. The galenic form according to claim 1, in which the chitosan has a degree of deacetylation of at least 50%.

3. The galenic form according to claim 1 to 2, in which, for 1 g of cationic polymer, the amount of metal ion(s) associated is from 1 to 300 mg, or in which, for 1 g of cationic polymer, the amount of metal ion(s) associated is from 20 to 200 mg.

4. The galenic form according to any one of claims 1 to 3, in which the particles have a diameter ranging from 0.01 µm to 1 mm.

5. The galenic form according to any one of claims 1 to 4, in which the particles are capable of specifically adsorbing the undesirable molecules chosen from the group comprising:
• domestic toxic substances;
• medicine residues or metabolites of medicines which are intentionally and/or accidentally ingested;
• medicine residues or the metabolites of the medicines most widely used in human or veterinary medicine that may be found in water;
• residual antibiotics, or in which the particles are capable of specifically adsorbing the residual antibiotics chosen from the group comprising quinolones, aminoglucosides, beta- lactam, macrolides, sulfamides, antitubercular agents and tetracyclines.

6. The galenic form according to claim 5, in which the undesirable molecules are residual antibiotics, optionally chosen from quinolones, aminoglucosides, betalactam, macrolides, sulfamides, antitubercular agents and tetracyclines, and the particles further contain at least one agent capable of inactivating said antibiotics, in particular an enzyme.

7. The galenic form according to any one of claims 1 to 6, in which the particles have a degree of crosslinking ranging from 0 to 100%.

8. The galenic form according to any one of claims 1 to 7, in which the particles are encapsulated in pectin beads, or in which the particles are encapsulated in beads made of zinc pectinate or calcium pectinate.

9. The galenic form according to claim 8, in which the concentration of pectin is from 5 to 95% by weight, relative to the total weight of the galenic form, and that of the cationic polymer-metal ion(s) association is from 5 to 95% by weight, relative to the total weight of the galenic form.

10. The galenic form according to anyone of claims 8 to 9, in which the pectin beads are spherical in shape and have a diameter ranging from 0.2 to 3 mm.

11. The galenic form according to any one of claims 1 to 10, in which the particles, optionally encapsulated in pectin beads, are coated with:
- a cationic polymer chosen from the group comprising polyethyleneimines, polylysines, polyarginines, DEAE dextran and chitosan;
- a cellulosic polymer chosen from the group comprising hydroxypropylcellulose, hydroxyethylcellulose, hydroxymethylcellulose, hydroxypropylmethylcellulose, hydroxypropylmethylcellulose acetate succinate, hydroxypropylmethylcellulose phthalate, methylcellulose, ethylcellulose, cellulose acetate, cellulose acetate phthalate, cellulose acetate trimellitate, and sodium carboxymethylcellulose;
- a polymer of acrylic acid, of methacrylic acid, of ethyl acrylate, of ethyl acrylate, of methyl methacrylate and/or of ethyl methacrylate, chosen from the group comprising Eudragits®, in particular Eudragit® NE, RL and RS, Eudragit® L30D-55 and L100-55, Eudragit® L100, Eudragit® S and Eudragit® FS30D;
- a vinyl polymer chosen from the group comprising polyvinylpyrrolidone, vinyl acetate, vinyl acetate phthalate, vinyl acetate-crotonic acid, and ethylenevinyl acetate.

12. A method for preparing a galenic form according to any one of claims 1 to 11, in which:
*(a)* a cationic polymer that is chitosan is dissolved in an aqueous solution of a metal salt or of a mixture of metal salts, under pH conditions <7, advantageously at a pH of between 1 and 6.8, even more advantageously at a pH between 1.2 and 6, in such a way that the metal ions of said salt or said mixture of salts associate with said cationic polymer so as to form a complex;
*(b)* the solution obtained in *(a)* is subjected to spray-drying, so as to obtain particles of said complex;
*(c)* crosslinking of the particles obtained in step *(b)* is optionally carried out in an organic solvent in the presence of a crosslinking agent.

13. The galenic form according to any one of claims 1 to 11, as a medicine.

14. The galenic form according to one of claims 1 to 11, for use thereof as a medicine in order to treat undesirable effects linked to an imbalance of the intestinal flora and/or a colic following treatment with antibiotics.

15. The galenic form according to claim 14, in which the medicine is administered before, during or after the administration of an antibiotic.
